(19) Europäisches Patentamt
European Patent Office
Office européen des brevets

(11) Publication number: **0 279 475 B1**

(12) **EUROPEAN PATENT SPECIFICATION**

(45) Date of publication of patent specification: **15.01.92**

(51) Int. Cl.5: **C07C 225/10**, C07C 309/02, C07C 323/00, G03C 1/72

(21) Application number: **88200105.0**

(22) Date of filing: **08.02.88**

(54) **Benzophenone derivatives.**

(30) Priority: **17.02.87 GB 8703606**

(43) Date of publication of application:
**24.08.88 Bulletin 88/34**

(45) Publication of the grant of the patent:
**15.01.92 Bulletin 92/03**

(84) Designated Contracting States:
**AT BE CH DE ES FR GB GR IT LI LU NL SE**

(56) References cited:
**EP-A- 0 209 831**

(73) Proprietor: **WARD BLENKINSOP AND COMPANY LIMITED**
**Halebank Factory Lower Road**
**Widnes Cheshire WA8 8NS(GB)**

(72) Inventor: **Green, Peter Nicholl**
**8 Ouickswood Drive**
**Woolton Liverpool L25 4TR(GB)**
Inventor: **Green, William Arthur**
**26 Ballantree Road**
**Liverpool L18 6JO(GB)**

(74) Representative: **Tuijn, Jan Warnaar et al**
**Shell Internationale Research Maatschappij B.V., Patents, Licensing & Trade Marks Division, P.O. Box 302**
**NL-2501 CH The Hague(NL)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to benzophenone derivatives, and their use as photoinitiators, in particular in the preparation of u.v.-cured lacquers.

A wide range of substrates, including metal, wood, paper, board and plastics materials are commonly coated with u.v.-cured lacquer films. Well known examples of coated articles include glossy magazines, books, food wrappers and drinks cans. The lacquers are generally formed by coating the substrates with a polymerisable composition containing a photoinitiator, and then curing the applied composition by exposing it to ultra violet light.

Compositions for the production of u.v.-cured lacquers normally contain a mixture of a lipid soluble prepolymer, a lipid soluble monomer and a lipid soluble photoinitiator. A co-initiator such as N-methyl diethanolamine is sometimes also present. Details of such compositions are given in "U.V. and E.B. curing formulations for printing inks, coatings and paint" edited by R. Holman and published in 1986 by SITA-Technology, U.K. Whilst such formulations can produce excellent u.v.-cured films on a variety of substrates, the monomers employed are often skin irritants, and sometimes it is difficult to achieve the desired composition viscosity without the aid of organic solvents. These problems can be overcome if an aqueous composition is employed, but the number of photoinitiators known to be suitable for use in such compositions is very small. Furthermore, such photoinitiators are in general expensive to manufacture because they cannot be made from readily available starting materials.

To be suitable for use in an aqueous composition for the production of u.v.-cured lacquers, a photoinitiator must be highly water soluble over a wide pH range, fast acting, colourless and, preferably, odourless.

One photoinitiator which is used commercially in the production of u.v.-cured lacquers is 4-benzoyl-N,N,N- trimethyl benzene methanaminium chloride. This compound is highly water soluble over a wide pH range, fast acting and colourless. However, lacquers prepared using the compound possess an undesirable amine-like odour. Furthermore, the compound is expensive to manufacture. Thus it is prepared from 4-methylbenzophenone by reaction firstly with a chlorinating agent to afford 4-chloromethylbenzophenone, and secondly with trimethylamine. 4-Methylbenzophenone may, itself, readily be prepared from toluene and benzoylchloride by a Friedel-Crafts reaction.

We have now found certain benzophenone derivatives which are suitable for use as photoinitiators in aqueous compositions for the production of u.v.-cured lacquers, which are odourless, and which may be prepared conveniently and cheaply from readily available starting materials.

The present invention provides a compound of the general formula

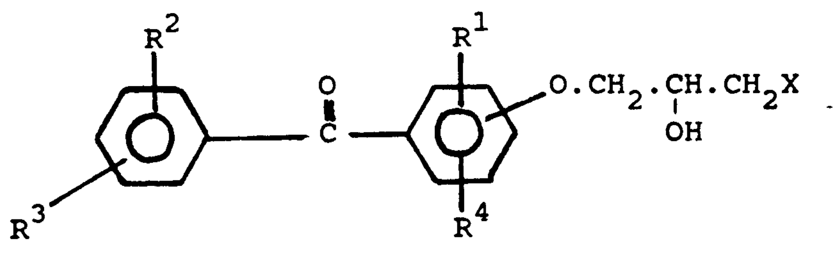

(I)

in which X represents $-SO_3H$ or an alkali metal salt thereof, or $NR^5R^6R^{7\,+}A^-$ in which $R^5$ represents an alkyl or benzyl group, each of $R^6$ and $R^7$ independently represents an alkyl group, and $A^-$ represents one equivalent of an anion; and each of $R^1$, $R^2$, $R^3$ and $R^4$ independently represents a hydrogen or halogen atom, a hydroxy group, a C(1-6) alkyl, alkoxy or alkylthio group, or a group of formula $-O.CH_2.CH(OH)CH_2X$, in which X has the meaning given above.

Preferably $R^1$ represents a C(1-6)alkyl group such as a methyl group or, more preferably, a hydrogen atom.

Preferably each of $R^2$, $R^3$ and $R^4$ independently represents a hydrogen atom, a chlorine atom, a hydroxy group, a methyl, methoxy or methylthio group, or a group of formula $-O.CH_2.CH(OH)CH_2\overset{+}{N}(CH_3)_3\bar{Cl}$. More preferably two of $R^2$, $R^3$ and $R^4$ represents a hydrogen atom. Especially preferred are compounds in which $R^2$, $R^3$ and $R^4$ all represent hydrogen atoms.

If $R^5$ represents an alkyl group, this preferably has up to 20 carbon atoms, for example $R^5$ may be a cetyl group, but more preferably $R^5$ has up to 6, especially up to 4 carbon atoms. $R^6$ and $R^7$ each preferably have up to 6, especially up to 4, carbon atoms. Most preferably, each of $R^5$, $R^6$ and $R^7$

represents a methyl group.

$A^-$ may represent one equivalent of a divalent ion such as sulphate, but is preferably a monovalent ion, for example halide or a sulphonate such as methane sulphonate. Most preferably $A^-$ is a chloride or bromide ion.

In one preferred group of compounds of the general formula I, X represents an $-SO_3Na$ group.

The invention further provides a process for the preparation of a compound of the general formula I, which comprises reacting a compound of the general formula

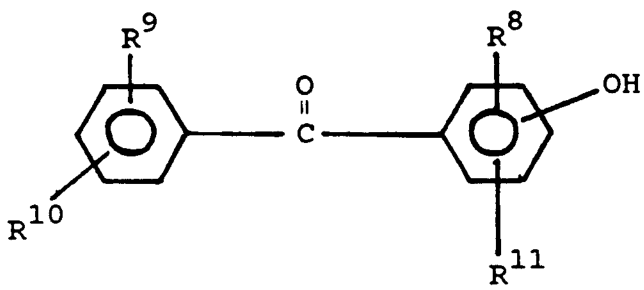

(II)

in which each of $R^8$, $R^9$, $R^{10}$ and $R^{11}$ independently represents a hydrogen or halogen atom, a hydroxy group, or a C(1-6) alkyl, alkoxy or alkylthio group, with a compound of the general formula

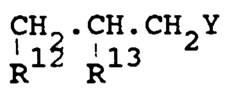

(III)

in which Y represents one of the groups X above or represents a halogen atom, and either $R^{12}$ is a leaving group and $R^{13}$ is a hydroxy group or $R^{12}$ and $R^{13}$ together represent an oxygen atom, in the presence of a base; and if Y represents a halogen atom, reacting the resultant product with an alkali metal sulphite, or with a compound of formula $NR^5R^6R^7$.

A leaving group $R^{12}$ is preferably a halogen, especially chlorine, atom.

The reaction is preferably carried out in the presence of a suitable solvent, for example an alkoxyalkanol or an alkanol, preferably a C(1-3) alkanol such as propan-2-ol. The reaction temperature is preferably in the range of from 40 to 120°C, especially 60 to 100°C, reflux temperature often being convenient. Suitable bases for use in the process include alkali metal (e.g. sodium and potassium) alkoxides and hydroxides. The number of moles of the compounds II and III reacting together will of course depend on the number of hydroxy groups in the compound II it is desired to replace with the group $-O.CH_2-CH(OH).CH_2X$.

The compounds of the general formula II may be prepared by methods analogous to known methods. For example, they may be prepared from the appropriate benzoylchlorides and phenols by a Friedel-Crafts reaction.

It is a particular advantage of compounds of the present invention that they can be synthesised conveniently and cheaply from readily-available starting materials. Of particular note in this respect is the compound 2-hydroxy-3-(4-benzoylphenoxy)-N,N,N-trimethyl-1-propanaminium chloride. This compound may be prepared in high yield from the readily available starting materials 4-hydroxybenzophenone and glycidyl trimethylammonium chloride.

Further in accordance with this invention there is provided an aqueous photocurable composition comprising an aqueous solution of at least one polymerisable prepolymer and, as photoinitiator, a compound of formula I, which can conveniently be present in a concentration between 0.5 and 10% by weight of the total formulation. The invention also extends to the use of such a composition for the production of u.v.-cured lacquer films, and to the films so produced.

Lacquer films may be prepared by exposing the aforementioned aqueous photocurable composition to u.v. light in a manner readily apparent to those skilled in the art.

Suitable photopolymerisable prepolymers include acrylated polyurethanes, polyesters and epoxides, for example those manufactured by Lankro Chemicals Limited, Eccles, U.K. and their water thinnable acrylated polyurethane prepolymer RCP 2702 (Trade Mark) is strongly recommended. This can be diluted with up to 30% by weight water before phase separation occurs.

A photocurable composition according to the invention may also if desired contain a water-soluble coinitiator. Particularly suitable coinitiators are water-soluble tertiary amines, for example triethanolamine,

N,N-dimethyl-ethanolamine or N-methyl-diethanolamine, such coinitiators can conveniently be present in a concentration between 0.5 and 10% by weight of the total formulation.

The following Examples illustrate the invention.

EXAMPLE 1
2-Hydroxy-3-(4-benzoylphenoxy)-N,N,N-trimethyl-1-propanaminium chloride

4-Hydroxybenzophenone (49.5g) and glycidyl trimethylammonium chloride (49.5g, 30% excess) together with propan-2-ol (250 mls) were stirred and brought to reflux whereupon the pH was adjusted to 9 by the addition of a few drops of ethanolic sodium ethoxide solution (3% w/v). The resultant pale brown solution was refluxed overnight followed by the addition of charcoal (0.2g) and Kieselguhr (0.2g). Hot filtration, followed by concentration under vacuo gave a viscous pale yellow syrup to which propan-2-ol (15 mls) followed by acetone (250 mls) was added. Subsequent cooling in an ice bath for twenty four hours resulted in the formation of a mass of white crystals. Filtration followed by washing (5% propan-2-ol/acetone, 20 mls × 2) and drying at 50 °C gave the crude title compound.

The off white solid (77.9g; m.p. 99 to 104 °C) was dissolved in a mixture of propan-2-ol (50 mls) and acetone (50 mls) and after the addition of acetone (250 mls) the mixture was stood in an ice bath overnight. Filtration, followed by washing (10% propan-2-ol/acetone, 25 mls × 2) and drying at 50 °C gave the title compound (69.35g; yield = 75.4%) as a white amorphous crystalline solid of melting point equal to 105.5 - 107 °C.

Analysis:-

Calcd. for $C_{19}H_{24}ClNO_3.1H_2O$
C 62.01; H 7.12; Cl 9.64; N 3.81
Found:
C 61.83; H 7.18; Cl 9.70; N 3.78

An aqueous solution of this compound has an absorption maximum at 293 nm with an extinction coefficient (1%, 1cm) of 436.

EXAMPLE 2
Sodium 2-hydroxy-3-(4-benzoylphenoxy)propane sulphonate

A solution of sodium hydroxide (3.3g) in water (5 mls) was added to a solution of sodium 3-chloro-2-hydroxypropane sulphonate (14.73g) in water (30 mls) and the resultant solution added to a solution of 4-hydroxybenzophenone (9.9g) in ethanol (50 mls). After stirring under reflux for 18 hours, the pH of the pale yellow solution was brought to 4 via the addition of 4N. hydrochloric acid and after cooling in an ice bath for several hours the crude title compound was filtered and twice washed with ethanol (20 ml portions) and dried at 50 °C. giving a pale brown sandy solid (11.58g) of melting point equal to 320-323 °C.

Recrystallisation with charcoaling from a mixture of ethanol (50 mls) and water (15 mls) followed by filtration, washing and drying gave sodium 2-hydroxy-3-(4-benzoylphenoxy) propane sulphonate (9.84g; yield = 55%) as a pale brown sandy solid of melting point equal to 316-318 °C.

Analysis:-

Calcd for $C_{16}H_{15}NaO_6S$
C 53.63; H 4.22; S 8.95; Na 6.42
Found:
C 53.57; H 4.24; S 8.75; Na 6.69

An aqueous solution of this compound has an absorption maximum at 294 nm with an extinction coefficient (1%, 1cm) of 467.

EXAMPLE 3
2-Hydroxy-3-(3-benzoylphenoxy)-N,N,N-trimethyl-1-propanaminium chloride

3-Hydroxybenzophenone (9.9g) and glycidyl trimethylammonium chloride (9.1g) together with propan-2-ol (60mls) were stirred under reflux for 6 hours. After charcoaling and hot filtration, the filtrate was concentrated under vacuo and a mixture of acetone (200mls) and water (1ml) added. The solution was

cooled in an ice-bath for 1 hour prior to filtration and washing with acetone (20mls × 2) and the resultant white solid was dried in an air oven at 50 °C prior to recrystallising to constant melting point. Four recrystallisations were required, using a solvent containing propan-2-ol (15%), ethanol (20%) and acetone (65%). The resultant title compound (4.29g; yield = 24.5%) is a white crystalline solid of melting point equal to 168-169.5 °C.

Analysis:

Calcd. for $C_{19}H_{24}ClNO_3$
C 65.24; H 6.91; N 4.00; Cl 10.14
Found
C 65.01; H 6.92; N 4.08; Cl 10.35
An aqueous solution of this compound has an absorption maximum at 258nm with an extinction coefficient (1%, 1cm) of 461.

EXAMPLE 4
2-Hydroxy-3-(2-benzoylphenoxy)-N,N,N-trimethyl-1-propanaminium chloride

2-Hydroxybenzophenone (9.9g) and glycidyl trimethylammonium chloride (9.1g) together with propan-2-ol (60mls) were stirred and refluxed overnight. After charcoaling and hot filtration, the filtrate was concentrated under vacuo and a mixture of acetone (200mls) and water (1ml) added. The solution was cooled in an ice bath for 1 hour prior to filtration and washing with acetone (20mls × 2) and the resultant brown solid was dried in an air oven at 50 °C prior to recrystallising to constant melting point. Five recrystallisations were required using a solvent containing propan-2-ol (18%), ethanol (10%) and acetone (72%). The resultant title compound (2.66g; yield = 15.2%) is a white crystalline solid of melting point equal to 166-167 °C.

Analysis;-

Calc. for $C_{19}H_{24}ClNO_3$
C 65.24; H 6.91; N 4.00; Cl 10.14
Found
C 65.00; H 6.98; N 4.01; Cl 10.37
An aqueous solution of this compound has an absorption maximum at 257 nm with an extinction coefficient (1%; 1cm) of 368.

EXAMPLE 5
2-Hydroxy-3-(2-benzoyl-5-methoxyphenoxy)-N,N,N,-trimethyl-1-propanaminium chloride

Glycidyl trimethylammonium chloride (3.04g) in water (1.3mls) was added to a stirred solution of 2-hydroxy-4-methoxybenzophenone (4.56g) in propan-2-ol (50mls) and the mixture refluxed overnight. After charcoaling and hot filtration, the filtrate was concentrated under vacuo and a mixture of acetone (50mls) and propan-2-ol (5mls) added. The mixture was cooled in an ice bath for 1 hour prior to filtration and washing with acetone (20mls × 2) and the resultant solid was dried in an air oven at 50 °C prior to dissolving in water (50mls) and extracting with chloroform (10mls × 3). The aqueous portion was concentrated under vacuo and the residue twice recrystallised from a mixture of propan-2-ol (50%) and acetone (50%). The resultant title compound (2.49g; yield = 32.7%) is a white crystalline solid of melting point equal to 192-193.5 °C.

Analysis:

Calc. for $C_{20}H_{26}ClNO_3.0.25H_2O$
C 62.49; H 6.95; N 3.64; Cl 9.22
Found
C 62.50; H 6.95; N 3.65; Cl 9.37
An aqueous solution of this compound has an absorption maximum at 250nm with an extinction coefficient (1%, 1cm) of 340.

EXAMPLE 6

2-Hydroxy-3-(4-(4-methylbenzoyl)phenoxy)-N,N,N-trimethyl-1-propanaminium chloride

4-Hydroxy-4′-methylbenzophenone (10.6g) and glycidyl trimethylammonium chloride (9.14g) together with propan-2-ol (60mls) were stirred and refluxed overnight. After charcoaling and hot filtration, the filtrate was concentrated under vacuo prior to dissolving in water (100mls) and extracting with toluene (20mls × 3). The aqueous portion was concentrated under vacuo and the residue thrice recrystallised from a mixture of propan-2-ol (30%), water (1.5%) and acetone (68.5%). The resultant title compound (8.7g; yield = 47.7%) is a white crystalline solid of melting point equal to 203.5-204.5°C.

Analysis:-

Calc. for $C_{20}H_{26}ClNO_3$
C 66.01; H 7.20; N 3.84; Cl 9.74
Found
C 65.88; H 7.19; N 3.83; Cl 9.86
An aqueous solution of this compound has an absorption maximum at 290nm with an extinction coefficient (1%, 1cm) of 529.

EXAMPLE 7

2-Hydroxy-3-(4-(4-chlorobenzoyl)phenoxy-N,N,N-trimethyl-1-propanaminium chloride hemihydrate

4-Chloro-4′-hydroxybenzophenone (11.67g) and glycidyl trimethylammonium chloride (9.14g) together with propan-2-ol (70mls) were stirred and refluxed overnight. After charcoaling and hot filtration, the filtrate was concentrated under vacuo prior to dissolving in warm water, acidifying to pH = 4 and filtering. The filtrate was extracted with chloroform (10mls × 2) and the aqueous portion concentrated under vacuo. The residue was thrice recrystallised from a mixture of propan-2-ol (39%), water (2%) and acetone (59%). The resultant title compound (2.65g; yield = 13.4%) is a white crystalline solid of melting point equal to 220-222°C.

Analysis;-

Calc. for $C_{19}H_{23}Cl_2NO_3.O.5H_2O$
C 58.02; H 6.15; N 3.56; Cl 18.03
Found
C 58.42; H 5.96; N 3.65; Cl 18.17
An aqueous solution of this compound has an absorption maximum at 293nm with an extinction coefficient (1%. 1cm) of 486.

EXAMPLE 8

2-Hydroxy-3-(4-benzoyl-2,6-dimethylphenoxy)-N,N,N-trimethyl-1-propanaminium chloride hemihydrate

3,5-Dimethyl-4-hydroxybenzophenone (8.6g) and glycidyl trimethylammonium chloride (6.96g) together with propan-2-ol (60mls) were stirred and refluxed overnight. After charcoaling and filtration, the filtrate was concentrated under vacuo prior to dissolving in water (50mls). The solution was extracted with toluene (10mls × 5) and the aqueous portion concentrated under vacuo. The residue was thrice recrystallised from a mixture of propan-2-ol (14%) and acetone (86%). The resultant title compound (2.05g; yield = 13.9%) is a white crystalline solid of melting point equal to 137-140°C.

Analysis:-

Calc. for $C_{21}H_{28}ClNO_3.O.5H_2O$
C 65.18; H 7.55; N 3.62; Cl 9.16
Found
C 64.95; H 7.55; N 3.67; Cl 9.54
An aqueous solution of this compound has an absorption maximum at 273nm with an extinction coefficient (1%; 1cm) of 353.

EXAMPLE 9
2-Hydroxy-3-(4-methylthiobenzoyl)phenoxy)-N,N,N-trimethyl-1-propanaminium chloride hydrate

4-Hydroxy-4′-methylthiobenzophenone was prepared via reaction between 4-methylthiobenzoyl chloride and anisole in the presence of aluminium chloride followed by demethylation with hydrobromic acid in the presence of a mixture of acetic acid and acetic anhydride.

4-Hydroxy-4′-methylthiobenzophenone (7.39g) and glycidyl trimethylammonium chloride (5.54g) together with propan-2-ol (60mls) were stirred and refluxed overnight. After charcoaling and hot filtration, the filtrate was concentrated under vacuo prior to dissolving in warm water (50mls) and acidifying to pH = 4 with 2N hydrochloric acid. Filtration, followed by extraction of the filtrate with chloroform (10mls × 2) and concentration of the aqueous portion under vacuo gave a creamy solid which was thrice recrystallised from a mixture of propan-2-ol (33%), water (1%) and acetone (66%). The resultant title compound (2.38g); yield = 19.29%) is a white crystalline solid of melting point equal to 204.5-206.5 °C).

Analysis:-

Calc. for $C_{20}H_{26}ClNO_3S.O.75H_2O$
C 58.66; H 6.40; N 3.42; Cl 8.65; S 7.83
Found
C 58.64; H 6.39; N 3.47; Cl 8.92; S 7.86
An aqueous solution of this compound has an absorption maximum at 318nm with an extinction coefficient (1%, 1cm) of 510.

EXAMPLE 10
3,3′-[1,1′-Carbonylbis((1,4-phenylene)bis oxy)]-bis(2-hydroxy-N,N,N-trimethylpropanaminium dichloride)-monohydrate

4,4′-Dihydroxybenzophenone (10.7g) and glycidyl trimethylammonium chloride (18.3g) together with propan-2-ol (60mls) were stirred and refluxed overnight. After charcoaling and filtering, the filtrate was concentrated under vacuo to a sticky solid to which ethanol (30mls) followed by acetone (150mls) were added. After standing for 2 hours, filtration followed by drying the residue at 50 °C the crude title compound.

The white solid was dissolved in ethanol (30mls) and acetone (100mls) added. After standing for 2 hours, filtration followed by washing with acetone (20mls × 2) and drying at 50 °C gave white crystals (12.52g) of melting point equal to 234-238 °C.

Analysis:-

Calc. for $C_{25}H_{38}Cl_2N_2O_5.1H_2O$
C 56.07; H 7.53; Cl 13.24; N 5.23
Found
C 56.43; H 7.51; Cl 13.41; N 5.16
An aqueous solution of this compound has an absorption maximum at 293nm with an extinction coefficient (1%, 1cm) of 436.

EXAMPLE 11
Disodium 3,3′-[1,1′-Carbonylbis((1,4-phenylene)bis oxy)]bis(2-hydroxypropanesulphonate)

Sodium 3-chloro-2-hydroxypropanesulphonate (29.47g) and 4,4′-dihydroxybenzophenone (10.7g) were added to a stirred solution of ethanolic sodium ethoxide (prepared by reacting sodium (2.53g) with absolute ethanol (150mls)) and the mixture was refluxed for 3 hours whereupon water (75mls) was added and the mixture refluxed for a further 3 hours. After cooling in an ice bath for 12 hour, filtration followed by washing with acetone (20mls × 2) and drying in an air oven at 50 °C, gave a white solid which was recrystallised 5 times from a mixture of water (50%) and ethanol (50%). The resultant title compound (5.28g; yield = 9.8%) is a white crystalline solid of metling point greater than 360 °C.

Analysis:-

Calc. for $C_{19}H_{20}Na_2O_{11}S_2$
C 42.69; H 3.77; S 12.00; Na 8.60
Found
C 42.92; H 3.81; S 11.70; Na 8.53
An aqueous solution of this compound has an absorption maximum at 298nm with an extinction coefficient (1%, 1cm)) of 454.

EXAMPLE 12
Comparision of the efficiency of the compounds given in Examples 1 to 11 with a prior art photoinitiator

Photocurable compositions were prepared by mixing Lankro's pre-polymer RCP 2702 (Trade Mark) (3.1g), water (1.75g), N-methyl diethanolamine (0.15g) and a photoinitiator (0.00043M) until homogeneous liquids were obtained. These compositions were coated onto glass microscope slides using a number two Bar coater and the resultant slides were exposed to UV light in a single lamp "Minicure" (Trade Mark) apparatus using a conveyor belt speed of 20 rpm for the minimum exposure times required to produce the maximum pencil hardness (ASTM-D3363-74) which the fully cured films were capable of producing, ie, 3H hardness.

| PHOTOINITIATOR | MINIMUM EXPOSURE TIME (SECS) | FILM COMMENTS |
|---|---|---|
| 1 | 0.24 | Colourless, good gloss, odourless |
| 2 | 0.20 | Colourless, good gloss, odourless |
| 3 | 0.39 | Colourless, good gloss, odourless |
| 4 | 0.29 | Colourless, good gloss, odourless |
| 5 | 0.39 | Colourless, good gloss, odourless |
| 6 | 0.34 | Colourless, good gloss, odourless |
| 7 | 0.34 | Colourless, good gloss, odourless |
| 8 | 0.29 | Colourless, good gloss, odourless |
| 9 | 0.24 | Colourless, good gloss, odourless |
| 10 | 0.49 | Colourless, good gloss, odourless |
| 11 | 0.59 | Colourless, good gloss, odourless |
| Comparative A | 0.24 | Colourless, good gloss, amine like odour |

The comparative A photoinitiator is 4-benzoyl-N,N,N-trimethylbenzenemethanaminium chloride.

The results clearly demonstrate that the compounds according to the invention are highly suitable as photoinitiators for use in aqueous compositions for the production of u.v.-cured lacquers, and possess the advantage of affording an odourless lacquer.

## Claims

1. A compound of the general formula

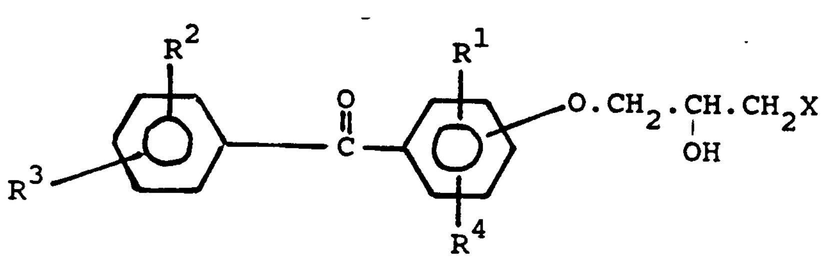

(I)

in which X represents $-SO_3H$ or an alkali metal salt thereof, or $NR^5R^6R^{7+}A^-$ in which $R^5$ represents an alkyl or benzyl group, each of $R^6$ and $R^7$ independently represents an alkyl group, and $A^-$ represents one equivalent of an anion; and each of $R^1$, $R^2$, $R^3$ and $R^4$ independently represents a hydrogen or halogen atom, a hydroxy group, a C(1-6) alkyl, alkoxy or alkylthio group, or a group of formula $-O.CH_2.CH(OH)CH_2X$, in which X has the meaning given above.

**2.** A compound as claimed in claim 1, in which $R^1$ represents a hydrogen atom.

**3.** A compound as claimed in claim 1 or 2, in which each of $R^2$, $R^3$ and $R^4$ independently represents a hydrogen atom, a chlorine atom, a hydroxy group, a methyl, methoxy or methylthio group, or a group of formula $-O.CH_2.CH(OH).CH_2N(CH_3)_3^+ Cl^-$.

**4.** A compound as claimed in claim 3, in which two of $R^2$, $R^3$ and $R^4$ represent hydrogen atoms.

**5.** A compound as claimed in claim 4, in which each of $R^2$, $R^3$ and $R^4$ represents a hydrogen atom.

**6.** A compound as claimed in any one of claims 1 to 5, in which X represents a group of formula $-NR^5R^6R^{7+}A^-$ in which each of $R^5$, $R^6$ and $R^7$ represents a methyl group.

**7.** A compound as claimed in any one of claims 1 to 6, in which $A^-$ represents a halide ion.

**8.** A compound as claimed in claim 1 or claim 2, in which X represents an $-SO_3Na$ group.

**9.** A process for the preparation of a compound as claimed in claim 1, which comprises reacting a compound of the general formula

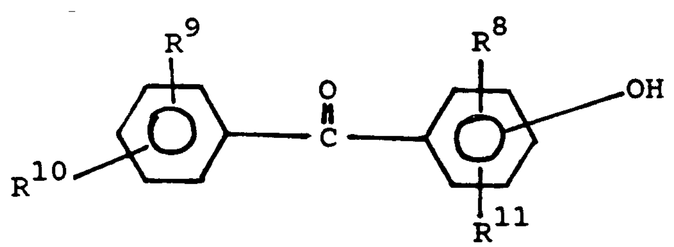

(II)

in which each of $R^8$, $R^9$, $R^{10}$ and $R^{11}$ independently represents a hydrogen or halogen atom, a hydroxy group, or a C(1-6) alkyl, alkoxy or alkylthio group, with a compound of the general formula

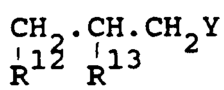

(III)

in which Y represents one of the groups X as defined in claim 1 or represents a halogen atom, and either $R^{12}$ is a leaving group and $R^{13}$ is a hydroxy group or $R^{12}$ and $R^{13}$ together represent an oxygen atom, in the presence of a base; and if Y represents a halogen atom, reacting the resultant product with an alkali metal sulphite, or with a compound of formula $NR^5R^6R^7$.

**10.** An aqueous photocurable composition comprising an aqueous solution of at least one polymerisable prepolymer and, as photoinitiator, a compound as claimed in any one of claims 1 to 8.

**11.** A method of producing a u.v.-cured lacquer film, which comprises exposing a composition as claimed in claim 10 to ultra violet light.

**12.** Lacquer films when prepared according to the method of claim 11.

## Revendications

**1.** Un composé de la formule générale

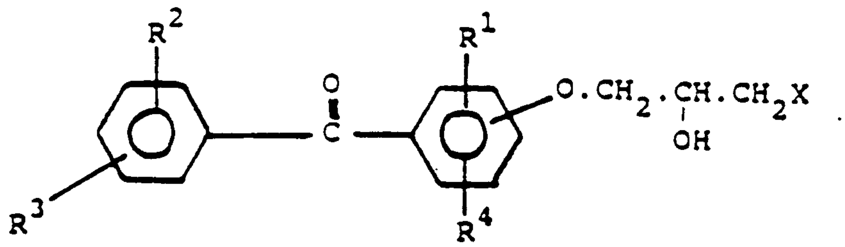

(I)

dans laquelle X représente $-SO_3H$ ou un de ses sels de métaux alcalins, ou $NR^5R^6R^{7+}A^-$ dans lequel $R^5$ représente un groupe alkyle ou benzyle, chacun des $R^6$ et $R^7$ représente indépendamment un groupe alkyle, et $A^-$ représente un équivalent d'un anion ; et chacun des $R^1$, $R^2$, $R^3$ et $R^4$ représente indépendamment un atome d'hydrogène ou d'halogène, un groupe hydroxy, un groupe alkyle, alcoxy ou alkythio en $C_1$ à $C_6$, ou bien un groupe de formule $-O.CH_2.CH(OH)CH_2X$ dans laquelle X a la signification spécifiée ci-dessus.

**2.** Un composé tel que revendiqué dans la revendication 1, dans lequel $R^1$ représente un atome d'hydrogène.

**3.** Un composé tel que revendiqué dans la revendication 1 ou 2, dans lequel chacun des $R^2$, $R^3$ et $R^4$ représente indépendamment un atome d'hydrogène, un atome de chlore, un groupe hydroxy, un groupe méthyle, méthoxy ou méthylthio, ou bien un groupe de formule $-O.CH_2.CH(OH)CH_2N^+(CH_3)Cl^-$.

**4.** Un composé tel que revendiqué dans la revendication 3, dans laquelle deux des $R^2$, $R^3$ et $R^4$ représentent des atomes d'hydrogènes.

**5.** Un composé tel que revendiqué dans la revendication 4, dans lequel chacun des $R^2$, $R^3$ et $R^4$ représentent un atome d'hydrogène.

**6.** Un composé tel que revendiqué dans l'une quelconque des revendications 1 à 5, dans lequel X représente un groupe de formule $-NR^5R^6R^{7+}A^-$ dans laquelle chacun des $R^5$, $R^6$ et $R^7$ représente un groupe méthyle.

**7.** Un composé tel que revendiqué dans l'une quelconque des revendications 1 à 6, dans lequel $A^-$ représente un ion halogénure.

**8.** Un composé tel que revendiqué dans la revendication 1 ou la revendication 2, dans lequel X représente un groupe $-SO_3Na$.

**9.** Un procédé pour la préparation d'un composé tel que revendiqué dans la revendication 1, dans lequel on fait réagir un composé de la formule générale

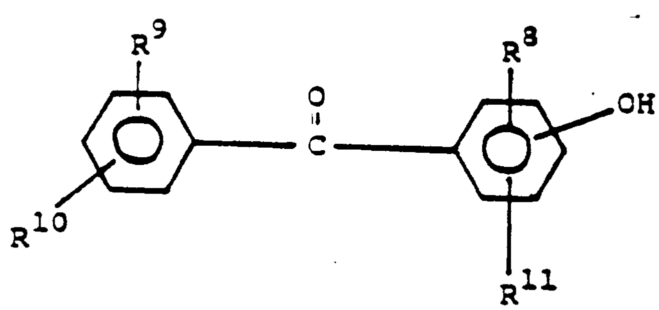

(II)

dans laquelle chacun des $R^8$, $R^9$, $R^{10}$ et $R^{11}$ représente indépendamment un atome d'hydrogène ou d'halogène, un groupe hydroxy, un groupe alkyle, alcoxy ou alkylthio en $C_1$ à $C_6$, avec un composé de la formule générale

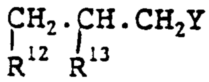

(III)

dans laquelle Y représente un des groupes X selon la revendication 1 ou bien représente un atome d'halogène, et soit $R^{12}$ est un groupe partant et $R^{13}$ est un groupe hydroxy, soit $R^{12}$ et $R^{13}$ représentent conjointement un atome d'oxygène, en présence d'une base ; et si Y représente un atome d'halogène, on fait réagir le produit résultant avec un sulfite de métal alcalin ou avec un composé de formule $NR^5R^6R^7$.

**10.** Un composition aqueuse photodurcissable comportant une solution aqueuse d'au moins un prépolymère polymérisable et, comme photo-initiateur, un composé tel que revendiqué dans l'une quelconque des revendications 1 à 8.

**11.** Une méthode pour la préparation d'une pellicule de résine durcie par ultraviolet, dans laquelle on expose une composition telle que revendiquée dans la revendication 10 à une radiation ultraviolette.

**12.** Pellicules de résine lorsqu'elles sont préparées selon la méthode de la revendication 11.

## Patentansprüche

**1.** Verbindung der allgemeinen Formel

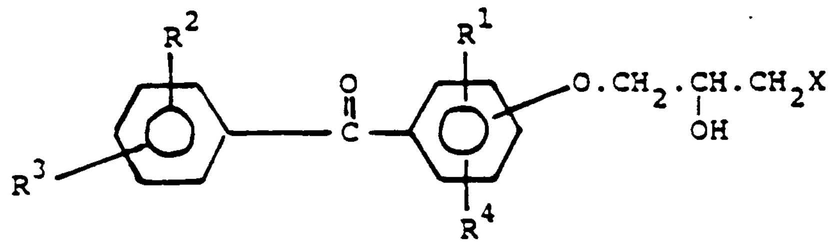

(I),

worin
X für $-SO_3H$ oder ein Alkalimetallsalz hievon steht oder für die Gruppe $NR^5R^6R^{7+}A^-$ steht, worin $R^5$ eine Alkyl- oder Benzylgruppe bedeutet, jeder der Reste $R^6$ und $R^7$ unabhängig voneinander eine Alkylgruppe darstellt und $A^-$ ein Äquivalent eines Anions bedeutet;
und jeder der Reste $R^1$, $R^2$, $R^3$ und $R^4$ unabhängig voneinander ein Wasserstoff- oder Halogenatom, eine Hydroxygruppe, eine C(1-6)-Alkyl-, -Alkoxy- oder -Alkylthiogruppe bedeutet oder eine Gruppe der Formel $-O.CH_2.CH(OH)CH_2X$ darstellt, worin X die zuvor angegebene Bedeutung aufweist.

**2.** Verbindung nach Anspruch 1, worin $R^1$ ein Wasserstoffatom darstellt.

**3.** Verbindung nach Anspruch 1 oder 2, worin jeder der Reste $R^2$, $R^3$ und $R^4$ unabhängig voneinander ein Wasserstoffatom, ein Chloratom, eine Hydroxygruppe, eine Methyl-, Methoxy- oder Methylthiogruppe oder eine Gruppe der Formel $-O.CH_2.CH(OH).CH_2N(CH_3)_3^+ Cl^-$ bedeutet.

**4.** Verbindung nach Anspruch 3, worin zwei der Reste $R^2$, $R^3$ und $R^4$ Wasserstoffatome bedeuten.

**5.** Verbindung nach Anspruch 4, worin jeder der Reste $R^2$, $R^3$ und $R^4$ ein Wasserstoffatom darstellt.

**6.** Verbindung nach einem der Ansprüche 1 bis 5, worin X eine Gruppe der Formel $-NR^5R^6R^{7+}A^-$ bedeutet, worin jeder der Reste $R^5$, $R^6$ und $R^7$ eine Methylgruppe darstellt.

**7.** Verbindung nach einem der Ansprüche 1 bis 6, worin $A^-$ ein Halogenidion bedeutet.

**8.** Verbindung nach Anspruch 1 oder 2, worin X eine $-SO_3Na$-Gruppe darstellt.

**9.** Verfahren zur Herstellung einer Verbindung nach Anspruch 1, welches ein Umsetzen einer Verbindung der allgemeinen Formel

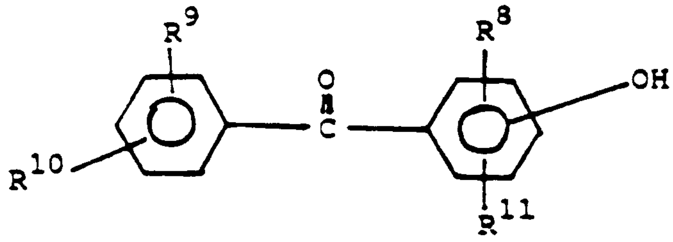

(II),

worin jeder der Reste $R^8$, $R^9$, $R^{10}$ und $R^{11}$ unabhängig voneinander ein Wasserstoff- oder Halogenatom, eine Hydroxygruppe oder eine C(1-6)-Alkyl-, -Alkoxy- oder -Alkylthiogruppe darstellt, mit einer Verbindung der allgemeinen Formel

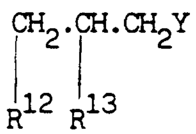

(III),

worin Y eine der Gruppen X, wie in Anspruch 1 definiert, darstellt oder ein Halogenatom bedeutet, und entweder $R^{12}$ eine Leavinggruppe ist und $R^{13}$ eine Hydroxygruppe bezeichnet oder $R^{12}$ und $R^{13}$ zusammen ein Sauerstoffatom darstellen, in Gegenwart einer Base umfaßt; und, falls Y ein Halogenatom darstellt, Umsetzen des erhaltenen Produktes mit einem Alkalimetallsulfit oder mit einer Verbindung der Formel $NR^5R^6R^7$.

**10.** Wäßrige photohärtbare Zusammensetzung, umfassend eine wäßrige Lösung wenigstens eines polymerisierbaren Präpolymers und, als Photoinitiator, eine Verbindung, wie in einem der Ansprüche 1 bis 8 beansprucht.

**11.** Verfahren zur Ausbildung eines UV-gehärteten Lackfilmes, welches ein Einwirkenlassen von Ultraviolettlicht auf eine Zusammensetzung nach Anspruch 10 umfaßt.

**12.** Lackfilme, hergestellt nach dem Verfahren von Anspruch 11.